# EUROPÄISCHE PATENTSCHRIFT

(11) **EP 1 600 100 B1**
(45) Veröffentlichungstag und Bekanntmachung des Hinweises auf die Patenterteilung: **23.04.2014**
(21) Anmeldenummer: 05001729.2
(22) Anmeldetag: 27.01.2005
(51) Int. Cl.: A61B 1/00, G02B 23/24, G02B 3/14

(54) **Zoomobjektiv für Endoskopiegeräte**
Zoom objective for an endoscope apparatus
Objectif zoom pour un appareil endoscopique

(30) Priorität: 27.05.2004 DE 102004026005
(43) Veröffentlichungstag der Anmeldung: 30.11.2005
(73) Patentinhaber: Invendo Medical GmbH, 69469 Weinheim (DE)
(72) Erfinder: Pauker, Fritz, 86438 Kissing (DE); Bob, Konstantin, Dr., 69469 Weinheim (DE); Viebach, Thomas, 86579 Diepoltshofen (DE)
(74) Vertreter: Winter, Brandl, Fürniss, Hübner, Röss, Kaiser, Polte - Partnerschaft

(56) Entgegenhaltungen:
- WO-A-97/36193
- DE-A1- 19 710 668
- DE-A1- 19 733 628
- FR-A- 2 634 287
- US-A- 3 161 718
- US-A- 4 286 839
- US-A- 4 331 388
- US-A- 5 406 417
- PATENT ABSTRACTS OF JAPAN Bd. 2000, Nr. 12, 3. Januar 2001 (2001-01-03) & JP 2000 249813 A (JAPAN SCIENCE & TECHNOLOGY CORP), 14. September 2000 (2000-09-14)
- PATENT ABSTRACTS OF JAPAN Bd. 004, Nr. 090 (P-017), 27. Juni 1980 (1980-06-27) & JP 55 052009 A (OLYMPUS OPTICAL CO LTD), 16. April 1980 (1980-04-16)

## Beschreibung

Die Erfindung bezieht sich auf ein Zoomobjektiv zur Verwendung beispielsweise in Endoskopen bzw. Endoskopiegeräten gemäß dem Oberbegriff des Patentanspruchs 1.

Endoskope sind zu einem wichtigen Hilfsmittel in der Technik und in der Medizin geworden, um kanalartige Hohlräume zu inspizieren, die ansonsten nur mit erheblichen Eingriffen zugänglich sind. Endoskope sind an ihrem Vorderende mit einer Beleuchtungseinrichtung und mit einer Optik zur visuellen Erfassung des davorliegenden Bereichs des Hohlraums ausgerüstet. Die am Vorderende des Endoskops erfasste, optische Information wird normalerweise entweder mittels einer Faseroptik durch das Endoskop nach hinten zu seinem Bedienungsende übertragen oder wird mittels eines optischen Sensorchips am Vorderende erfasst, durch eine elektrische Leitung durch das Endoskop nach hinten geleitet und auf einem Bildschirm sichtbar gemacht. Darüber hinaus ist eine Übertragung der am Vorderende des Endoskops erfassten Informationen mittels Funk zum Bedienungsende denkbar.

Die noch nicht veröffentlichte Patentanmeldung DE 102 54 609.6 beschreibt einen Endoskopkopf der mit einer Anzahl von Funktionseinheiten, wie beispielsweise Optik, Beleuchtungselemente, Spüldüsen und dergleichen ausgerüstet ist. Die Optik des Endoskopkopfs besteht im Wesentlichen aus einer kubischen Sensorchipkammer, einer darüber liegenden, durch eine Trennwand von der Sensor- oder Kamerachipkammer abgeteileten, zylindrischen Linsenkammer, die zumindest eine optische Linse bzw. ein Linsensystem aufnimmt, die unmittelbar in der Linsenkammer fixiert sind oder in Form einer vorgefertigten Patrone in die Kammer einführbar ist. In der DE 102 54 609.6 wird zwar darauf verwiesen, dass das Linsensystem auch zoombar ausgeführt sein kann, es werden jedoch keine weiterführenden Details beschrieben. Außerdem wird kein Hinweis darauf gegeben, wie ein solches zoombares Linsensystem realisiert werden kann.

Aufgrund der Fortschritte in der Digitalfotografie und der damit verbundenen graphischen Weiterverarbeitung von visuellen Informationen ist es möglich, ein gewonnenes Bild oder Ausschnitte davon mit Bildbearbeitungsprogrammen zu vergrößern. Somit könnte mit der oben genannten DE 102 54 609.6 zwar ein zoomfähiges Endoskop realisiert werden, jedoch beinhaltet diese Technologie folgende Nachteile.

Bei der Bildvergrößerung mittels feststehender Linsen und anschließender Nachbearbeitung (digitaler Zoom) wird nur ein Ausschnitt des Bildes vergrößert, d.h. die Anzahl der Bildpunkte aus denen sich das Bild zusammensetzt, bleibt gleich. Mit anderen Worten verschlechtert sich die Auflösung des Bildes und die Schärfe sowie die Bildqualität des vergrößerten Ausschnitts wird schlechter. Auch hier bieten gute Bildbearbeitungsprogramme die Funktion mittels Interpolation die ursprüngliche Auflösung beizubehalten, jedoch handelt es sich bei den rechnerisch hinzugewonnenen Bildinformationen nicht um reale visuelle Informationen, sondern um statistisch errechnete Bildpunkte. Details können somit zwar Schärfe vortäuschen, aber in Wirklichkeit verfälscht sein.

Somit bleibt bei der Vergrößerung von Bildausschnitten nur die Möglichkeit des optischen Zoomens, um ein scharfes und detailgetreues Bild zu erhalten. Bei Endoskopen stellt sich jedoch aufgrund der Miniaturbauweise das Problem, dass die herkömmlich in der Fotografie verwendeten Technologien, wie z.B. eine piezoelektrische Verstellung der Linsen, zur Veränderung der Brennweite nicht anwendbar sind, weil sie zuviel Platz in Anspruch nehmen, der in einem Endoskopkopf nicht vorhanden ist.

Die DE 197 33 628 A offenbart ein Zoomobjektiv entsprechend dem Oberbegriff des Anspruchs 1. DE 197 33 628 A offenbart einen Stellantrieb bestehend aus einem Führungszylinder, in weichem zwei axial verstellbare Kolben angeordnet sind. Die Verstellung der Kolben wird über jeweils in Axialrichtung vor und hinter den Kolben ausbildete Druckkammern bewirkt, die entsprechend einer gewünschten Stellung mit Fluid druckbeaufschlagt bzw. druckentspannt werden. In einer Ausführungsform ist der Stellantrieb Teil eines Endoskops, wobei die Kolben als Linsen ausgebildet sind.

Die US 4,286,839 richtet sich auf ein Lichtausrichtungs-Steuersystem (also eine Beleuchtungseinrichtung) mit einem Licht-Streulinsen-System. Dieses Linsensystem umfasst zwei axial bewegliche Linsen, deren jeweils voneinander abgewandte Linsenseiten federbeaufschlagt sind, d. h. die Federn gemäß der US 4,286,839 sind ausschließlich auf nur einer Seite jeder beweglichen Linse angeordnet, derart, dass die Linsen aufeinander zu vorgespannt sind.

Damit die Linsen nicht aufeinander zu liegen kommen, ist zwischen den Linsen eine mit Fluid befüllbare Druckkammer ausgebildet. Je nach Füllstand der Druckkammer werden die beiden Linsen entgegen der äußeren Federvorspannung voneinander wegbewegt (gedrückt).

Angesichts dieses Stands der Technik ist es eine Aufgabe der Erfindung, ein Zoomobjektiv für die Verwendung in einem Endoskop zu realisieren, das eine ausreichende Auflösung bei kleinem Bauraum ermöglicht.

### ZUSAMMENFASSUNG DER ERFINDUNG

Diese Aufgabe wird durch ein Zoomobjektiv mit den Merkmalen des Anspruchs 1 gelöst. Der Kern der Erfindung besteht prinzipiell darin, dass eine Anzahl von Linseneinheiten, von der mindestens eine Linseneinheit hydraulisch/pneumatisch verstellbar ist, in einem Linsenzylinder, im Wesentlichen senkrecht zu dessen Mittellinie angeordnet sind. Dieser Linsenzylinder ist vorzugsweise einstückig mit einem Endoskopkopf ausgebildet oder wird in diesen eingefügt.

Zwischen benachbarten Linseneinheiten ist jeweils eine Fluidkammer/ein Druckraum ausgebildet, an welche wahlweise Fluidleitungen angeschlossen sind. Die hydraulische Verstellung der mindestens einen Linseneinheit in der Axialrichtung des Linsenzylinders zur Brennweitenveränderung des Zoomobjektivs wird dadurch bewirkt, dass vorzugsweise inkompressibles Fluid in die entsprechenden Fluidkammern zugeführt bzw. aus den entsprechenden Fluidkammern abgeführt wird.

Ferner sind in den Fluidkammern Federn eingesetzt, welche die verstellbaren Linsen in einer Ausgangs- oder Konstruktionslage positionieren.

Ein wesentlicher Vorteil einer hydraulischen/pneumatischen Verstellung der Linseneinheiten liegt in dem geringen Platzbedarf in der unmittelbaren Umgebung des Zoomobjektivs im Endoskopkopf. Bisherige Zoomobjektive erfordern einen hohen Platzbedarf in der direkten Umgebung der Linsen zur Anordnung des Objektivantriebs. Der Vorteil der hydraulischen/pneumatischen Verstellung liegt darin, dass der Stellmotor nicht in der Umgebung der Linsen angeordnet sein muss, sondern an eine Stelle verlagert werden kann, wo Platzbedarf nur eine untergeordnete Rolle spielt. Die für eine solche Verstellung nötigen Fluidleitungen werden in dem Endoskopschaft eingebettet, in dem verglichen mit dem Endoskopkopf mehr Platz zur Verfügung steht.

Weitere vorteilhafte Ausgestaltungen der Erfindung sind Gegenstand der übrigen Patentansprüche.

### KURZBESCHREIBUNG DER ZEICHNUNGEN

Die Erfindung wird nachstehend anhand bevorzugter Ausführungsbeispiele bezugnehmend auf die beigefügten Zeichnungen genauer beschrieben, dabei ist in den Zeichnungen folgendes schematisch dargestellt:
Fig. 1 ist eine räumliche Darstellung eines ersten Ausführungsbeispiels des Zoomobjektivs der vorliegenden Erfindung;
Fig. 2 ist eine Seitenansicht des Zoomobjektivs aus Fig. 1;
Fig. 3 ist eine geschnittene Vorderansicht entlang des Schnitts A-A aus Fig. 2;
Fig. 4 stellt eine Ausführung einer Kolben-ZylinderEinheit gemäß einem ersten Ausführungsbeispiel dar;
Fig. 5 ist eine räumliche Darstellung eines zweiten Ausführungsbeispiels des Zoomobjektivs der vorliegenden Erfindung;
Fig. 6 ist eine Seitenansicht des Zoomobjektivs aus Fig. 5;

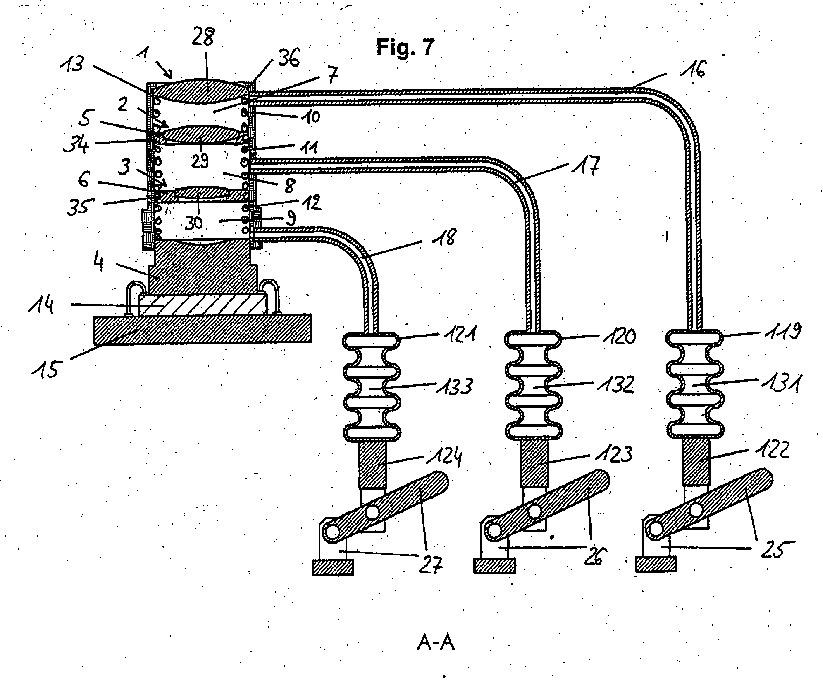

Fig. 7 ist eine geschnittene Vorderansicht entlang des Schnitts A-A aus Fig. 6;
Fig. 8 ist eine durch die Mittelachse des Zoomobjektivs geschnittene Vorderansicht eines weiteren Beispiels welches nicht Teil der Erfindung ist; und

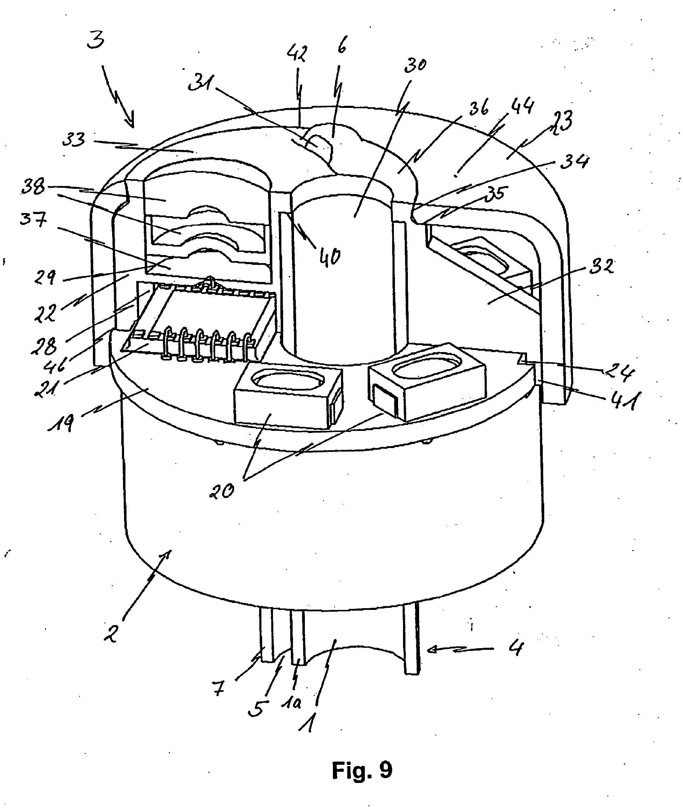

Fig. 9 ist ein Endoskopkopf eines Endoskopiegerätes vom Stand der Technik.

### DETAILLIERTE BESCHREIBUNG DER BEVORZUGTEN AUSFÜHRUNGSBEISPIELE

Im Wesentlich besteht ein Endoskop (nicht dargestellt) aus einem flexiblen Endoskopschaft (nicht dargestellt), an dessem Vorderende ein Endoskopkopf (nicht dargestellt) angefügt ist und an dessen anderem Ende (Bedienungsende) Bedienungs- und Informationsauswertungsgeräte (nicht dargestellt) angeschlossen sind. Dieser Endeskopkopf kann mit verschiedenen Einrichtungen und Werkzeugen, wie beispielsweise dem in dieser Erfindung vorgestellten Zoomobjektiv, bestückt werden. Die in dieser Beschreibung verwendeten Begriffe "Vorne" und "Hinten" beziehen sich auf die Verwendungsrichtung des Endoskops, somit ist das Vorderende des Endoskops jenes Ende, das bei der Verwendung in den Hohlraum eingeführt wird.

### (Erstes Ausführungsbeispiel)

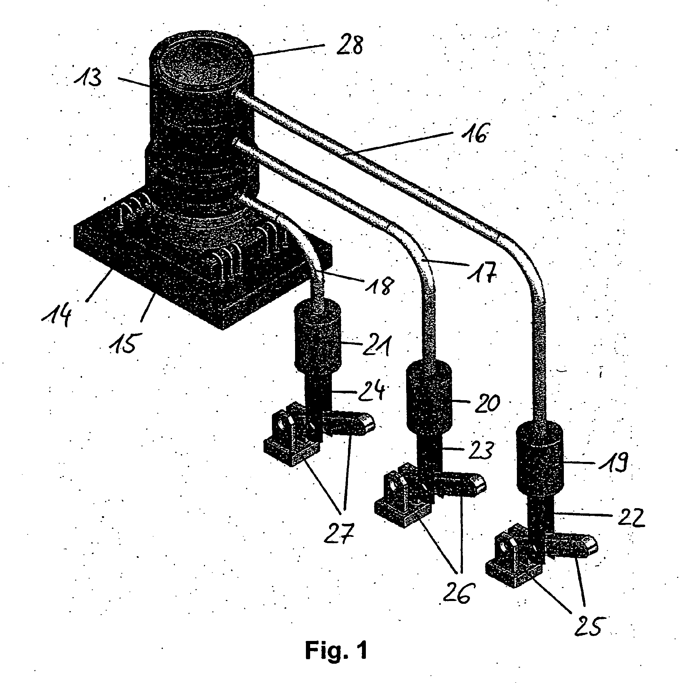

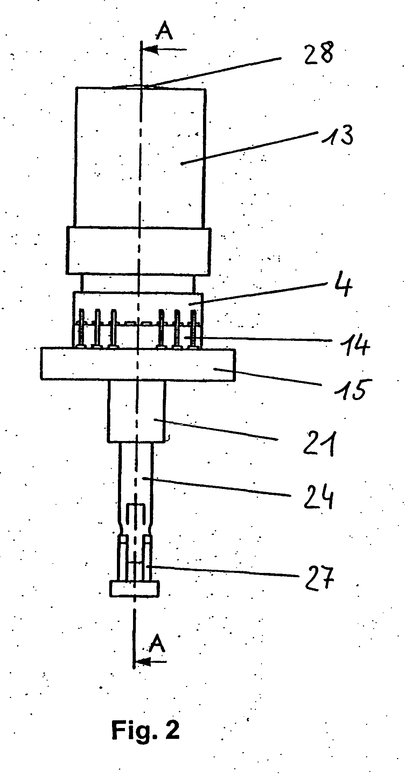

Anhand der Figuren 1 bis 3 wird das Zoomobjektiv zur Verwendung in einem Endoskopkopf (nicht dargestellt) gemäß einem ersten Ausführungsbeispiel näher beschrieben. Das Zoomobjektiv besteht aus einem im Wesentlichen rohrförmigen Linsenzylinder 13. Dieser ist entweder mit einen Endoskopkopf (nicht dargestellt) einstückig verbunden oder lösbar in diesem montiert.

Der Linsenzylinder 13 nimmt in dem vorliegenden Ausführungsbeispiel vier Linseneinheiten 1 - 4 auf. Die Linseneinheit 1 besteht aus einer Linse 28, welche am Vorderende des Linsenzylinders 13 in diesem, senkrecht zur Mittellinie des Linsenzylinders 13 angeordnet ist. Der Außendurchmesser der Linse 28 ist entsprechend dem Innendurchmesser des Linsenzylinders 13 angepasst, so dass der Linsenzylinder 13 nach Vorne fluiddicht verschlossen wird. Der Linsenzylinder 13 weist an seinem Vorderende einen Vorsprung 36 auf, der sich gleichmäßig von der Innenfläche des Linsenzylinders 13 nach Innen erstreckt. Somit ist der Innendurchmesser des Linsenzylinders 13 an der Stelle des Vorsprungs 36 größer als der Außendurchmesser der Linse 28, wodurch diese in dem Linsenzylinder 13 gehalten wird. Alternativ, wäre auch eine andere Möglichkeit denkbar, wie z.B. ein Presssitz, um ein Entfernen der Linse 28 aus der vorderen Öffnung des Linsenzylinders 13 zu unterbinden.

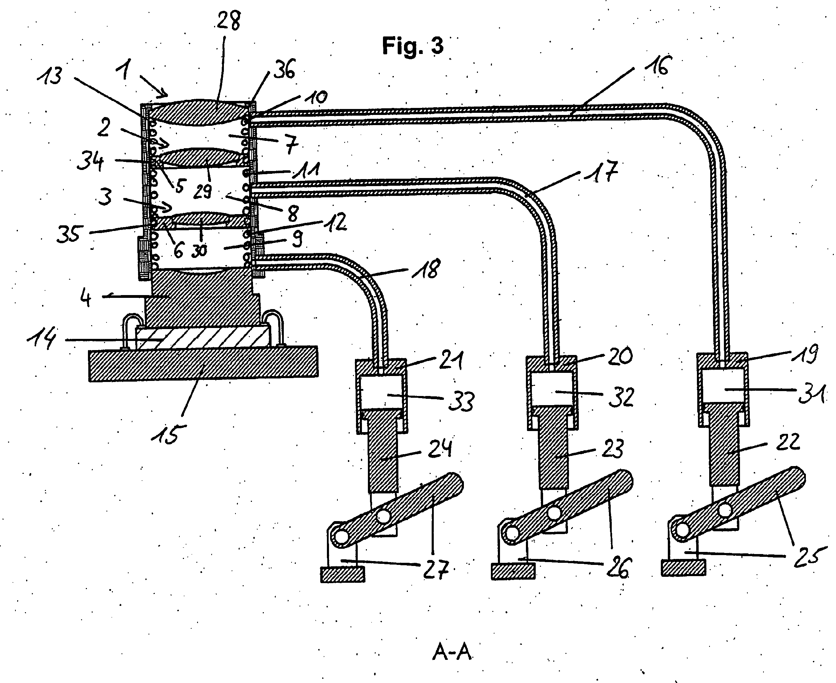

Die Linseneinheit 2 und die Linseneinheit 3 sind im Wesentlichen kreisförmig und jeweils senkrecht zur Mittellinie des Linsenzylinders 13 angeordnet. Sie bestehen jeweils aus einer Linse 29 und 30, einer Linsenfassung 5 und 6 sowie einem Dichtungsring 34 und 35. Die Linsenfassungen 5 und 6 sind ringförmig ausgeführt, wobei in deren mittigen kreisförmigen Öffnungen jeweils eine Linse 29 und 30 angeordnet ist. In der kreisförmigen Öffnung der Linsenfassungen 5 und 6 ist bezüglich der Längsrichtung des Linsenzylinders 13 ein Steg eingebracht, so dass der jeweilige Innendurchmesser der Linsenfassungen 5 und 6 auf der Vorderseite (oben in Fig. 3) im Wesentlichen gleich zum Außendurchmesser der jeweiligen Linsen 29 und 30 und auf der Hinterseite minimal kleiner als der Außendurchmesser der jeweiligen Linsen 29 und 30 ist. Die Linsen 29 und 30 können dann von Vorne in die entsprechenden Linsenfassungen 5 und 6 eingefügt werden und sind relativ zu den Linsenfassungen 5 und 6 genau positioniert, da sie an den Stegen anliegen. Dabei können die Linsen 29 und 30 beispielsweise durch einen Presssitz oder durch Kleben in der Linsenfassung gehalten werden. Die Außendurchmesser der Linsenfassungen 5 und 6 sind minimal kleiner als der Innendurchmesser des Linsenzylinders 13, so dass die Linsenfassungen 5 und 6 in der Axialrichtung des Linsenzylinders 13 bewegbar sind. In den peripheren Außenflächen der Linsenfassungen 5 und 6 sind jeweils ringförmige Nuten eingeformt, welche jeweils einen Dichtungsring 34 und 35 aufnehmen. Diese Dichtungsringe 34 und 35 dichten zwischen den peripheren Außenflächen der entsprechenden Linsenfassungen 5 und 6 und der Innenfläche des Linsenzylinders 13, so dass fluiddichte Fluidkammern/Druckräume 7 - 9 entstehen, die später genauer beschrieben werden.

Die hintere Öffnung des Linsenzylinders wird durch eine weitere Linseneinheit 4 verschlossen. Diese Linseneinheit hat auf seiner vorderen Hälfte die Form eines Zylinders, dessen Längsachse mit der Längsachse des Linsenzylinders 13 übereinstimmt. Dabei entspricht der Außendurchmesser dieser Zylinderform dem Innendurchmesser des Linsenzylinders 13. Die zylindrische Vorderseite der Linseneinheit 4 wird dann so in die hintere Öffnung des Linsenzylinder 13 eingefügt, dass dieser fluiddicht verschlossen ist. Des Weiteren weist die Linseneinheit 4 auf ihrer Vorderseite eine mittige konkave Mulde auf, durch die das am Vorderende des Objektivs eingefallene Licht gebeugt und an einen optischen Sensorchip 14 weitergegeben wird. Die hintere Hälfte der Linseneinheit 4 hat eine kubische Form, wobei dessen Seitenlängen in der Ebene senkrecht zur Längsachse des Linsenzylinders 13 der Größe des optischen Sensorchips 14 angepasst ist.

Dieser optische Sensorchip 14 ist mit seiner lichtempfindlichen Seite an der Hinterseite der Linseneinheit 4 angeordnet und wandelt visuelle Informationen in elektrische Signale um. Der optische Sensorchip 14 ist auf seiner dem Linsenzylinder abgewandten Seite auf einem Funktionsträger 15 montiert.

Durch die fluiddichte Anordnung der vier Linseneinheiten 1 - 4 entstehen zwischen den Linseneinheiten 1 - 4 die drei Fluidkammern 7 - 9, welche jeweils von zwei benachbarten Linseneinheiten 1 - 4 und der Innenfläche des Linsenzylinders 13 abgegrenzt werden.

An jede der Fluidkammern 7 - 9 ist eine flexible Fluidleitung 16 - 18 angeschlossen, die von den Fluidkammern 7 - 9 durch den Endoskopschaft (nicht dargestellt) nach hinten zum Bedienungsende des Endoskopschafts führen. Diese Fluidleitungen 16 - 18 sind vorteilhafterweise möglichst mittig im Endoskopschaft angeordnet, um eine Beeinflussung des Drucks des darin befindlichen Fluids möglichst gering zu halten. Genauer bedeutet dies, dass beim Einführen des Endoskopschafts in einen mehrfach stark gekrümmten Hohlraum, der Endoskopschaft ebenfalls stark gekrümmt wird, was zu einer Querschnittsverengung der in dem Endoskopschaft befindlichen Fluidleitungen 16 - 18, an den gekrümmten Stellen, führen kann. Diese Querschnittsverengung führt zu einer Druckschwankung des in den Fluidleitungen befindlichen Fluids, dabei können sich die Druckschwankungen in den einzelnen Fluidleitungen 16 - 18 unterscheiden, je nachdem, ob die entsprechende Fluidleitung 16 -18 in dem Endoskopschaft mehr zur Krümmungsaußen- oder -innenseite angeordnet ist. Dies erschwert eine genaue Justierung der Linseneinheiten, die später detaillierter beschrieben wird. Dieser Effekt der Querschnittsverengung wird je mehr abgeschwächt desto mittiger die Fluidleitungen in dem Endoskopschaft angeordnet sind. Zur Minderung dieser Druckbeeinflussing sind die Fluidleitungen 16 - 18 in dem vorliegenden Ausführungsbeispiel Vorteilhafterweise möglichst mittig im Endoskopschaft angeordnet.

In jede Fluidkammer 7 - 9 ist eine Feder 10 - 12 eingefügt, die in dem vorliegenden Ausführungsbeispiel als Spiralfeder ausgeführt und so angeordnet ist, dass deren Längsachse mit der Längsachse des Linsenzylinders 13 zusammenfällt. Zweckmäßigerweise ist der Außendurchmesser der Federn 10 - 12 minimal kleiner als der Innendurchmesser des Linsenzylinders 13, so dass die Bewegungen der Federn nicht gebremst oder blockiert werden, wenn diese zusammengedrückt werden oder sich ausdehnen. Die Enden der jeweiligen Federn 10 - 12 sind an den Linseneinheiten abgestützt und bilden eine ringförmige Auflagefläche mit diesen aus. Diese Federn können in einer gewünschten Art und Weise vorgespannt sein, um die verstellbaren Linseneinheiten 2 und 3 in einer Ausgangs- oder Konstruktionslage zu positionieren. Eine Montage des Objektivs wird dadurch vereinfacht, dass die Federn 10 - 12 zwei benachbarte Linseneinheiten voneinander beabstanden, so dass sich die empfindlichen Linsen nicht berühren können, wenn noch kein Fluid in die Fluidkammern 7 - 9 gefüllt ist. Des Weiteren sorgen die Federn 10 - 12 durch ihr gleichmäßiges Andrücken in der Ebene senkrecht zur Längsachse des Linsenzylinders 13 gegen die Linseneinheiten 2 und 3, für eine gleichmäßige Axialbewegung und vermeiden somit beim Bewegen ein Schrägstellen oder Verkanten der Linseneinheiten 2 und 3 in dem Linsenzylinder 13. Auf weitere Aspekte der Funktionsweise der Federn 10 - 12 wird später eingegangen.

An dem Bedienungsende jeder Fluidleitung 16 - 18 ist jeweils ein Zylinder 19 - 21 angeschlossen, in dem ein Kolben 22 - 24 gleitfähig eingefügt ist. Von der Außenfläche des Kolbens 22 - 24, die der Innenfläche des Zylinders 19 - 21 zugewandt ist und der Innenfläche des Zylinders 19 - 21 wird ein Aktuatorraum 31 - 33 abgegrenzt, der über die entsprechende Fluidleitung 16 - 17 mit der Fluidkammer 7 - 9 in Verbindung steht.

Die Fluidkammern 7 - 9, die Fluidleitungen 16 - 18 sowie die Aktuatorräume 31 - 33 sind jeweils mit einem möglichst inkompressiblen Fluid gefüllt, das, aufgrund des Vorhandenseins zwischen den Linseneinheiten 1 - 4, entsprechende optische Eigenschaften aufweisen muss, um die Lichtstrahlen in geeigneter Weise zu beugen.

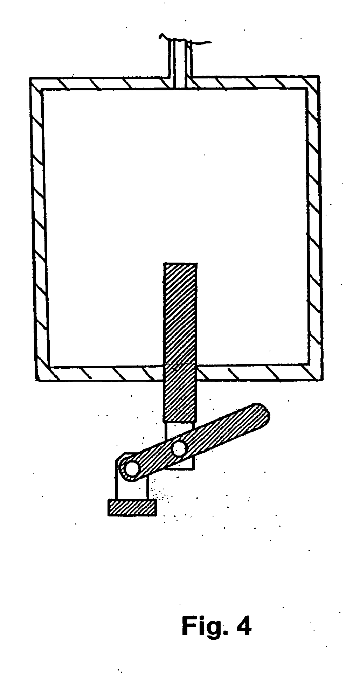

Bezüglich der Zylinder-Kolben-Einheit ist es vorteilhaft, wenn der Außendurchmesser des Kolbens 22 - 24 deutlich kleiner als der Innendurchmesser des Zylinders 19 - 21 ist, wie in Fig. 4 dargestellt. Auf diese Weise wird eine Untersetzung für die Bewegung der Kolben 22 - 24 realisiert, indem für eine bestimmte vorgegebene Druckänderung im jeweiligen Aktuatorraum 31 - 33 eine größere Kolbenbewegung erforderlich ist, verglichen mit der Kolbenbewegung, wenn der Innendurchmesser der Zylinder 19 - 21 gleich dem Außendurchmesser der Kolben 22 - 24 ist.

Die Kolben 22 - 24 werden jeweils von einem Aktuator 25 - 27 bewegt, der symbolhaft in den Figuren als Hebel dargestellt ist. Für die Aktuatoren können beispielsweise elektrische Schrittmotoren oder Magnetspulen zum Einsatz kommen .

### (Funktionsweise - erstes Ausführungsbeispiel)

Im Folgenden wird nun die Funktionsweise des Zoomobjektivs gemäß dem ersten Ausführungsbeispiel der vorliegenden Erfindung im Zoombetrieb beschrieben.

Soll beispielsweise in Fig. 3 die Linseneinheit 3 nach Vorne bewegt werden, wird der Kolben 22 durch den Aktuator 25 arretiert, der Aktuator 26 in Leerlauf geschaltet, so dass sich der Kolben 23 frei bewegen kann und der Kolben 24 wird durch den Aktuator 27 in den Zylinder 24 bewegt. Dadurch wird in dem Aktuatorraum 33 befindliches Fluid über die Fluidleitung 18 in die Fluidkammer 9 gedrückt, wodurch sich die Linseneinheit 3 entgegen der Federkraft der Feder 11 und unterstützt durch die Federkraft der Feder 12 nach Vorne bewegt. Durch das Zuführen von Fluid in die Fluidkammer 9 muss die gleiche Menge an Fluid aus der Fluidkammer 7 und/oder 8 gedrängt werden. Da der Kolben 22 arretiert ist, kann kein Fluid aus der Fluidkammer 7 über die Fluidleitung 16 in den Aktuatorraum 31 gedrängt werden. Somit wird Fluid gleichermaßen aus der Fluidkammer 8 über die Fluidleitung 17 in den Aktuatorraum 32 gedrückt, wie Fluid in die Fluidkammer 9 zugeführt wird. Dadurch wird der Kolben 23 in dem Zylinder 20 in Richtung nach Außen bewegt. Des Weiteren wird die Feder 11 zusammengedrückt und die Feder 12 dehnt sich aus.

Vorstehend wurde die Verstellung der Linseneinheit 3 beschrieben, indem der Kolben 22 arretiert wird, der Kolben 23 frei beweglich bleibt und der Kolben 24 in den Zylinder bewegt wird. Die gleiche Verstellung kann jedoch bewirkt werden, indem der Kolben 22 arretiert wird, der Kolben 24 frei beweglich bleibt und der Kolben 23 in dem Zylinder 20 in Richtung nach Außen bewegt wird. Des Weiteren können auch zwei der Kolben 22 - 24 frei beweglich bleiben und der dritte der Kolben 22 - 24 in dem entsprechenden Zylinder hinein- oder herausbewegt werden, um beide Linseneinheiten 2 und 3 gleichzeitig zu bewegen.

Auf diese Weise ergibt sich eine Vielzahl von Kombinationsmöglichkeiten, die Kolben 22 - 24 anzusteuern, um die gewünschte Verstellung der Linseneinheiten 2 und 3 zu erreichen. Da diese anderen Kombinationsmöglichkeiten nach dem gleichen Schema ablaufen, wie das oben Beschriebene, wird auf eine ausführliche Beschreibung aller Kombinationsmöglichkeiten verzichtet.

Auf diese Weise kann in dem vorliegenden Ausführungsbeispiel die Linseneinheit 2 und 3 zur Erreichung einer bestimmten Brennweite entsprechend über die Aktuatoren 25 - 27 bewegt werden. Dabei werden die Aktuatoren 25 - 27 von einer Steuerung (nicht dargestellt) angesteuert, um eine schnelle und präzise Positionierung der Linseneinheiten 2 und 3 zu erreichen.

Zu beachten ist bei der Ansteuerung der Aktuatoren 25 - 27 jedoch, dass die Steuerung ermöglicht, dass die Menge an in die Fluidkammern 7 - 9 zugeführtem Fluid, gleichermaßen aus den Fluidkammern 7 - 9 abgeführt werden muss. Das heißt, die Steuerung darf die Kolben 22 - 24 nicht derart steuern, dass beispielsweise einer der Kolben 22 - 24 in den Zylinder hineinbewegt wird, während die beiden anderen Kolben 22 - 24 arretiert bleiben.

Eine starke Krümmung der Fluidleitungen 16 - 18 kann zu einer Druckschwankung in den jeweiligen Fluidleitungen 16 - 18 und damit in den Fluidkammern 7 - 9 führen. Diese Beeinflussung der Linseneinheiten-Positionierung kann neben der passenden Anordnung der Fluidleitungen 16 - 18 im Endoskopschaft (nicht dargestellt) auch durch eine entsprechende Einstellung der Federkräfte der Federn 10 - 12 gemindert werden. Dabei sind die Federn 10 - 12 so anzupassen, dass deren Federkräfte so hoch sind, dass sich eine Linseneinheit 2 oder 3 erst ab einer bestimmten Druckdifferenz zwischen zwei benachbarten Fluidkammern 7 - 9 bewegt, welche höher ist als die Druckdifferenz, die bei der Krümmung des Endoskopschafts entsteht. Auf diese Weise kann eine präzisere Justierung der Linseneinheiten 29 und 30 erreicht werden, die nicht von der Krümmung des Endoskopschafts beeinflusst wird.

### (Zweites Ausführungsbeispiel)

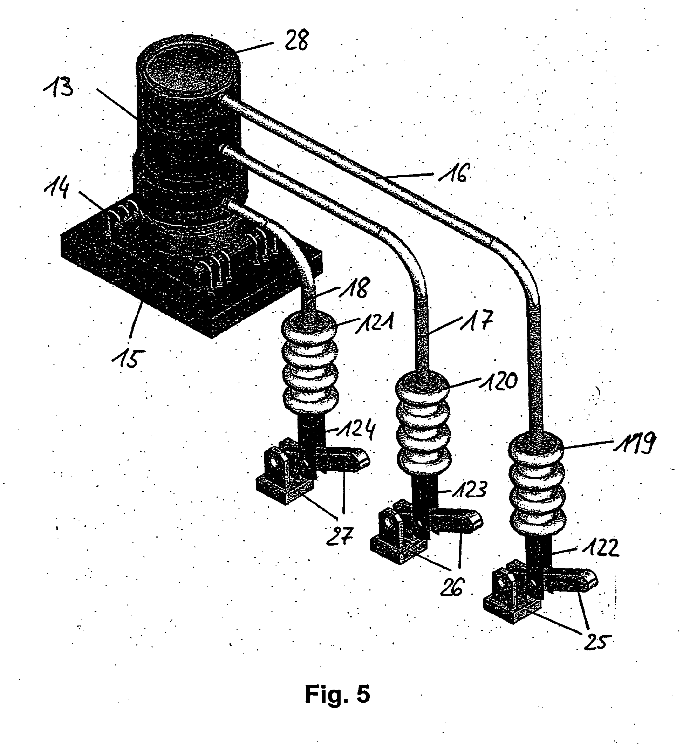

Anhand der Figuren 4 bis 6 wird das Zoomobjektiv zur Verwendung in einem Endoskopkopf (nicht dargestellt) gemäß einem zweiten Ausführungsbeispiel näher beschrieben. Diejenigen Bauteile in den Figuren 4 bis 6, die identisch zu denen aus den Figuren 1 bis 3 sind, haben die gleichen Bezugsnummern und deren Beschreibung wird im Folgenden weggelassen.

Insbesondere werden nun die Bauteile beschrieben, die sich vom ersten Ausführungsbeispiel unterscheiden.

Anstatt der Zylinder 19 - 21 des ersten Ausführungsbeispiels, sind auf der Bedienungsseite der Fluidleitungen 16 - 18, Balge 119 - 121 an die Fluidleitungen 16 - 18 angeschlossen. Die Innenräume der Balge 119 - 121 bilden Aktuatorräume 131 - 133 aus, die jeweils über die entsprechenden Fluidleitungen 16 - 18 mit den entsprechenden Fluidkammern 7 - 9 in Verbindung stehen. Auf der Seite der Balge 119 - 121, welche den Fluidleitungen 16 - 18 abgewandt ist, sind jeweils Schäfte 122 - 124 an die Außenseite der Balge 119 - 121 angeschlossen. Diese Schäfte 122 - 124 sind wie die Kolben 22 - 24 des ersten Ausführungsbeispiels mit Aktuatoren 25 - 27 verbunden.

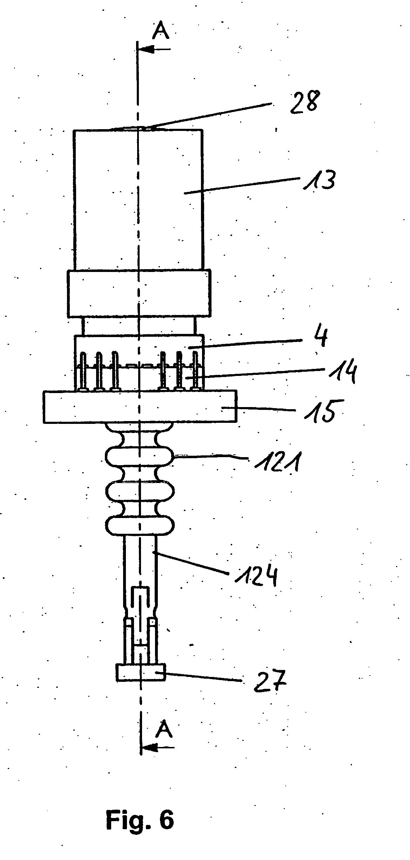

Wenn ein Schaft 122 - 124 in Fig. 6 nach Oben bewegt wird, wird der zugehörige Balg 119 - 121 zusammengedrückt und das Volumen des entsprechenden Aktuatorraums 131 - 133 wird verkleinert. Daher wird Fluid aus dem entsprechenden Aktuatorraum 131 - 133 über die zugehörige Fluidleitung 16 - 18 in die entsprechende Fluidkammer 7 - 9 gedrückt. Wenn umgekehrt ein Schaft 122 - 124 in Fig. 6 nach Unten bewegt wird, wird der zugehörige Balg 119 - 121 ausgedehnt und Fluid aus der zugehörigen Fluidkammer 7 - 9 angesaugt. Auf diese Weise wird die gleiche Wirkung wie durch die Kolben-Zylinder-Einheiten des ersten Ausführungsbeispiels erzielt. Auf eine genauere Funktionsweise des zweiten Ausführungsbeispiels wird aus diesem Grund nicht eingegangen, da sie, abgesehen vom Austausch der Kolben-Zylinder-Einheiten gegen die Balgeinheiten, identisch zu der des ersten Ausführungsbeispiels ist.

### (Erstes und zweites Ausführungsbeispiel)

Bei der Dimensionierung der Größe der Aktuatorräume 31 - 33 und 131 - 133 und der Anbringung der Fluidleitungen 16 - 17 ist zu verhindern, dass sich eine der Linseneinheiten 2 oder 3 soweit bewegen kann, dass eine der Fluidkammern 7 - 9 mit einer Fluidleitung 16 - 17 verbunden wird, die für die Ansteuerung einer anderen Fluidkammer 7 - 9 vorgesehen ist. Das heißt, die Fluidleitungen 16 - 17 sind bezüglich der Längsrichtung des Linsenzylinders 13 so an diesem anzuschließen, dass sich die Linseneinheiten 2 und/oder 3 in dem Linsenzylinder 13 ausreichend vor und zurück bewegen können, ohne dass beispielsweise bei einer Vorwärtsbewegung der Linseneinheit 3 die Fluidkammer 9 mit der Fluidleitung 17 und 18 verbunden wird. Für die Dimensionierung der Aktuatorräume 31 - 33 und 131 - 133 bedeutet dies, dass deren Volumen nur so groß gewählt wird, dass beispielsweise bei einer Vorwärtsbewegung der Linseneinheit 3 nicht die Fluidkammer 9 mit der Fluidleitung 17 und 18 verbunden wird. Alternativ kann dies auch durch entsprechend festgelegte Grenzwerte der Steuerung, welche die Aktuatoren 25 - 27 ansteuert, verhindert werden.

### (Weiteres Beispiel)

In einem weiteren Beispiel, welches nicht Teil der Erfindung ist, haben jene Bauteile, die identisch zu denen des ersten und zweiten Ausführungsbeispiels sind, gleiche Bezugsnummern und deren Beschreibung wird weggelassen.

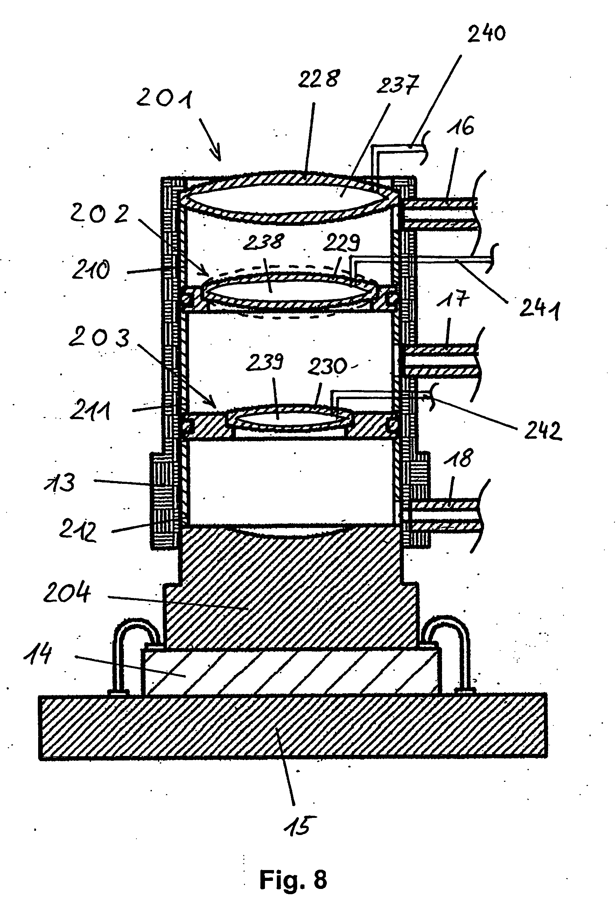

Anhand der Figur 8 wird das Zoomobjektiv zur Verwendung in einem Endoskopkopf (nicht dargestellt) gemäß dem Beispiel näher beschrieben.

Der Linsenzylinder 13 nimmt auch in dem vorliegenden Beispiel vier Linseneinheiten 201 - 204 auf. Dabei unterscheiden sich die Linseneinheiten 201 - 203 von den Linseneinheiten 1 - 3 des ersten und zweiten Ausführungsbeispiels darin, dass die Linsen 228 - 230 flexible Linsen sind. Diese flexiblen Linsen 228 - 230 sind so aufgebaut, dass sie jeweils einen Hohlraum 237 - 239 im Inneren ausbilden. Der Aufbau der Linsen 228 - 230 kann beispielsweise so ausgeführt werden, dass zwei runde schalenförmige Membrane an ihren Pheripherien so luftdicht zusammengefügt (z.B. geklebt) werden, dass sich die Membrane in entgegengesetzten Richtungen nach Außen wölben und im Inneren der zusammengefügten Membrane jeweils einen Hohlraum 237 - 239 ausbilden. Die auf diese Art und Weise zusammengefügten Membrane bilden im dritten Ausführungsbeispiel die Linsen 228 - 230, deren Hohlräume 237 - 239 jeweils mit einem Medium gefüllt sind. Als Füllmedium der Hohlräume ist bevorzugterweise ein Gas vorgesehen, es ist aber auch die Verwendung eines inkompressiblen oder leicht kompressiblen Fluids denkbar, je nachdem, welche optischen Eigenschaften erreicht werden sollen. Darüber hinaus können die Hohlräume mit Hohlraumzuleitungen 240 - 242 verbunden sein, die in Figur 8 schematisch dargestellt sind. Über diese Hohlraumzuleitungen 240 - 242 können durch Zuführen von Medium in die Hohlräume oder durch Abführen von Medium aus den Hohlräumen die Krümmungsradien der Linsen 228 - 230 bestimmt werden. Dazu wird beispielsweise Medium über die Hohlraumzuleitungen 240 - 242 zugeführt, um die Krümmungsradien der Membrane der entsprechenden Linsen 228 - 230 zu verkleiner bzw. die Membrane der Linsen nach Außen gerichtet mehr zu wölben, wie durch eine gestrichelte Linie in Fig. 8 für die Linse 229 schematisch dargestellt. Entsprechend werden durch Abführen von Medium aus den Hohlräumen 237 - 239 die Krümmungsradien der Membrane der entsprechenden Linsen 228 - 230 vergrößert bzw. die Membrane der Linsen nach Innen gerichtet verändert. Durch diese Art und Weise der Veränderung bzw. Einstellung der Krümmungssradien mittels der Hohlräume 237 - 239 und der Hohlraumzuleitungen 240 - 242 wurde eine Möglichkeit zur Brennweitenveränderung der einzelnen Linsen 228 - 230 beschrieben, die für jede einzelne Linse 228 - 230 unabhängig von den anderen Linsen 228 - 230 durchführbar ist. Über die Veränderung der Brennweite der einzelnen Linsen 228 - 230 ist ebenfalls die Gesamtbrennweite des Zoomobjektivs veränderbar, da diese von der Brennweite der einzelnen Linsen abhängt.

Die Linseneinheit 204 dieses Beispiels ist identisch zur Linseneinheit 4 des ersten und zweiten Ausführungsbeispiels.

Vorstehende beschriebene Druckdifferenzveränderung zwischen den Hohlräumen 237 - 239 und der Umgebung der Linsen 228 - 230, sprich der Fluidkammern 7 - 9, kann aber nicht nur durch Druckveränderung in den Hohlräumen 237 - 239 realisiert werden, sondern auch/zusätzlich durch eine Druckveränderung in den Fluidkammern 7 - 9.

Dazu können die Linseneinheiten 202, 203 in dem Beispiel so ausgeführt sein, dass sie in der Axialrichtung des Linsenzylinders 13 nicht verschiebbar beabstandet sind. Diese Fixierung kann beispielsweise durch einen Preßsitz der Linseneinheiten 202, 203 im Linsenzylinder 13 realisiert werden. In diesem Ausführungsbeispiel ist jedoch zwischen den Linseneinheiten 201 - 204 jeweils eine Hülse 210 - 212 koaxial zum Linsenzylinder 13 angeordnet. Dabei ist der Außendurchmesser der Hülsen 210 - 212 minimal kleiner als der Innendurchmesser des Linsenzylinders 13. Die Hülsen 210 - 212 dienen dazu, die Linseneinheiten 202, 203 in der Axialrichtung in einem vorherbestimmten Abstand zueinander und zu den Linseneinheiten 201, 204 anzuordnen. Um eine Verbindung der Fluidleitungen 16 - 17 und der Fluidkammern 7 - 9 zu schaffen, sind die Hülsen 210 - 212 an den Stellen, wo die Fluidleitungen 16 - 17 in die Fluidkammern 7 - 9 münden, jeweils mit Bohrungen versehen, deren Durchmesser groß genug ist, um die Fluidein- und -ausströmung in die Fluidkammern 7 - 9 nicht zu behindern.

Durch das Zu- und Abführen von Fluid in die Fluidkammern 7 - 9 können die Krümmungsradien der Membrane der Linsen 228 - 230 ebenfalls verändert werden. Dabei werden beispielsweise durch Zuführen von Fluid über die Fluidleitung 17 in die Fluidkammer 8 vor allem die Krümmungsradien der Linsen 229 und 230 verändert. Die Möglichkeit der Krümmungsradiuseinstellung der Linsen mittels Druck-/Volumenveränderung des Fluids in den Fluidkammern 7 - 9 kann mit der Krümmungsradiuseinstellung mittels Druck-/Volumenveränderung des Mediums in den Hohlräumen 237 - 239 kombiniert werden oder unabhängig voneinander verwendet werden. Des Weiteren kann bei der Möglichkeit der Krümmungseinstellung mittels Druck-/Volumenveränderung des Fluids in den Fluidkammern 7 - 9 auf die Hohlraumzuleitungen 240 - 242 verzichtet werden. In diesem Fall wären die Hohlräume 237 - 239 mit einer festen Menge eines kompressiblen Mediums gefüllt. Dieses könnte beispielsweise die Hohlräume 237 - 239 derart druckbeaufschlagen, dass es dem Fluid in den Fluidkammern 7 - 9 entgegenwirkt, so dass die Form der Linsen 228 - 230 eine gewisse Spannkraft zu Formbeibehaltung aufweist.

Die in diesem Beispiel vorgestellte flexible Linse 228 - 230 muss aber nicht notwendigerweise mit einer feststehenden Linseneinheit 202, 203 kombiniert werden, sondern kann auch mit hydraulisch verstellbaren Linseneinheiten 2 und 3, die im ersten und zweiten Ausführungsbeispiel der Erfindung beschrieben wurden, ausgeführt werden.

Die Fluidkammern 7 - 9 können demnach dazu verwendet werden, die Linseneinheiten 2 und 3 hydraulisch in der Axialrichtung des Linsenzylinders zu verschieben, wie im ersten und zweiten Ausführungsbeispiel beschrieben oder können auch zur Krümmungsradiuseinstellung der Membrane der Linsen 228 - 230 verwendet werden.

Außerdem besteht auch die Möglichkeit die nicht verschiebbare axiale Beabstandung der Linseneinheiten 201 - 204 des dritten Ausführungsbeispiels dadurch zu realisieren, dass auf die Fluidleitungen 16 - 18, die Hülsen 210 - 212 und die Bohrungen im Linsenzylinder 13 zum Einführen der Fluidleitungen 16 - 18 verzichtet wird. Bei einer solchen Ausführung wären die Fluidkammern 7 - 9 mit einem Medium gefüllt, das entweder inkompressibel ist oder kompressibel und druckbeaufschlagt, sodass die Linseneinheiten 202 und 203 in vorherbestimmten Positionen axial beabstandet sind.

### (Weitere Variationsmöglichkeiten)

Schließlich sei darf hingewiesen, dass die hier vorliegende Beschreibung und die beigefügten Figuren nur beispielhafter Natur sind und keinesfalls dazu dienen sollen, die Erfindung auf die hier vorgestellten Ausführungen zu begrenzen. Die Erfindung lässt eine Vielzahl von Anwendungsmöglichkeiten und Modifikationen zu ohne den Kern der Erfindung und deren Rahmen zu verlassen. Der Kern der Erfindung wird durch die Ansprüche definiert.

Einige Modifikationen werden im Folgenden genannt:
Im ersten und zweiten Ausführungsbeispiel wurde die hydraulische Verstellung der Linseneinheiten (2, 3) dadurch bewirkt, dass Fluid in Fluidkammern 7 - 9 zu- bzw. aus ihnen abgeführt wurde. Diese hydraulische Verstellung der Linseneinheiten 2, 3 kann aber auch durch Zu- bzw. Abführen von Fluid in bzw. aus Fluidkammern bewirkt werden, welche von Kolben/Zylinder-Einheiten ausgebildet werden. Genauer bedeutet dies, dass von den Innenwänden eines Zylinders und der Kolbenfläche eines in den Zylinder eingefügten Kolbens, die dem Innenraum des Zylinders zugewandt ist, diese Fluidkammer definiert wird. Die Kolben/Zylinder-Einheiten könnten dabei in der Wandung des Linsenzylinder 13, oder in der Umgebung der Wandung des Linsenzylinders 13 angeordnet sein. Ein Wirkende einer Kolben/Zylinder-Einheit ist mit einer zu verstellenden Zylindereinheit 2, 3 funktionell verbunden und das andere Wirkende der Kolben/Zylinder-Einheit ist entweder mit einer anderen Zylindereinheit 2, 3 oder mit dem Linsenzylinder 13 verbunden. Die funktionelle Verbindung des einen Endes der Kolben/Zylinder-Einheit kann dadurch bewirkt werden, dass dieses Ende direkt mit einer zu verstellenden Zylindereinheit 2, 3 verbunden ist oder für den Fall, dass die Kolben/Zylinder-Einheit außerhalb des Linsenzylinders 13 angeordnet ist, dieses Ende durch magnetische Kraft oder einen durch die Wandung des Linsenzylinders 13 hindurchreichenden Verbindungsstifts bewirkt wird, wobei bei letzterer Möglichkeit, die für den Verbindungsstift notwendige Öffnung mit einer den Verbindungsstift umgebenden Manschette abgedichtet sein müsste. Die Verstellung der verstellbaren Linseneinheiten 2, 3 wird nach dem gleichen Prinzip wie beim ersten und zweiten Ausführungsbeispiel durchgeführt, wobei entsprechend die Fluidkammern über Fluidleitungen 16 - 18 mit den Aktuatorräumen 31 - 33 verbunden sind.

In den Ausführungsbeispielen ist der Linsenzylinder 13 rohrförmig, er kann jedoch auch eine andere Formen, wie z.B. eine Ellipsenform, aufweisen. Die darin befindlichen Linseneinheiten müssten dann ebenfalls dieser geänderten Geometrie angepasst sein.

Die Erfindung wurde in den vorliegenden Ausführungsbeispielen jeweils mit drei Linseneinheiten ausgestattet, jedoch ist die Erfindung nicht darauf zu begrenzen und kann mit einer Vielzahl an Linseneinheiten realisiert werden.

In den beschriebenen Ausführungsbeispielen weist eine Linseneinheit jeweils eine Linse auf. Dies ist allerdings beispielhaft und eine Linseneinheit kann eine Vielzahl an konvexen oder konkaven Linsen aufweisen.

Außerdem ist die Erfindung nicht wie in dem ersten und zweiten Ausführungsbeispiel auf die Verwendung von Spiralfeder begrenzt, sondern kann auch mit anderen Federn ausführt werden.

In den beschriebenen Ausführungsbeispielen wurde bevorzugterweise ein möglichst inkompressibles Fluid in die Fluidkammern und ein Gas in die Hohlräume der Linsen gefüllt. Jedoch ist die Erfindung nicht darauf zu beschränken, sondern sowohl Gas als auch Fluid kann sowohl in den Hohlräumen der Linsen als auch in den Fluidkammern verwendet werden, je nachdem, welche optischen Eigenschaften das Linsensystem des Zoomobjektivs aufweisen soll.

Die Übermittlung der visuellen Informationen vom Endosokopkopf zum Bedienende ist nicht wie in dem ersten und zweiten Ausführungsbeispiel beschrieben, auf die Verwendung eines optischen Sensorchips beschränkt, sondern kann z.B. auch mit Hilfe einer Faseroptik durchgeführt werden.

Bevorzugterweise beträgt die Abmessung des Zoomobjektivs in Längsrichtung maximal 20 mm und dessen Durchmesser maximal 10 mm.

Ein Zoomobjektiv zur Verwendung in Endoskopiegeräten, in dem eine Vielzahl an Linseneinheiten in einem Linsenzylinder, senkrecht zu dessen Mittellinie, angeordnet sind. Aus der Vielzahl an Linseneinheiten kann zumindest eine Linseneinheit in der Axialrichtung des Linsenzylinders hydraulisch verstellbar ausgeführt sein, um die Brennweite des Zoomobjektivs zu verändern. Außerdem kann zusätzlich die Brennweite von einzelnen Linsen der Linseneinheit durch pneumatische Formänderung der Linsen verändert werden, um somit die Gesamtbrennweite des Zoomobjektivs zu verändern.

## Patentansprüche

1. Zoomobjektiv vorzugsweise für Endoskopiegeräte mit:
einer Linsenaufnahme (13)
einer Anzahl an Linseneinheiten (1 - 4) jeweils bestehend aus zumindest einer Linse (28 - 30), die in der Linsenaufnahme (13), im Axialabstand zueinander angeordnet sind, wobei die Brennweite des Zoomobjektivs veränderbar ist, indem der Abstand zwischen der Linseneinheiten (1 - 4) hydraulisch und/oder pneumatisch verändert wird, wofür jeweils zwischen benachbarten Linseneinheiten (1 - 4) ein Druckraum zur hydraulischen/pneumatischen Veränderung der Brennweite ausgebildet ist, wodurch jeweils zu beiden Seiten jener Linseneinheiten (2, 3), die verstellbar sind, Druckräume (7 - 9) ausgebildet sind, **dadurch gekennzeichnet, dass** in jedem der Druckräume zwischen den Linseneinheiten (1 - 4) ein Federelement (10-12) angeordnet ist, die unter einer bestimmten Vorspannungen gehalten sind um jene Linseneinheiten (2, 3), die verstellbar sind, in einer Ausgangs- oder Konstruktionslage zu positionieren.

2. Zoomobjektiv vorzugsweise für Endoskopiegeräte gemäß
einem der vorstehenden Ansprüche,
**dadurch gekennzeichnet, dass**
mindestens eine der Linseneinheiten (2, 3) in der Axialrichtung der Linsenaufnahmeeinheit (13) hydraulisch verschiebbar ist, um somit die Brennweite des Zoomobjektivs zu verändern.

3. Zoomobjektiv vorzugsweise für Endoskopiegeräte gemäß einem der vorstehende Ansprüche, **dadurch gekennzeichnet, dass** an die Druckräume (7 - 9) zwischen benachbarten Linseneinheiten (1 -4) Fluidleitungen (16-18) angeschlossen sind.

4. Zoomobjektiv vorzugsweise für Endoskopiegeräte gemäß Anspruch 3,
**dadurch gekennzeichnet, dass**
eine Veränderung der Druckdifferenz zwischen den Druckräumen (7 - 9) zur Brennweitenverstellung des Zoomobjektivs dadurch bewirkt wird, dass Fluid in ausgewählte Druckräume (7 - 9) zugeführt bzw. aus anderem ausgewählten Druckräumen (7 - 9) abgeführt wird.

5. Zoomobjektiv vorzugsweise für Endoskopiegeräte gemäß einem der vorstehenden Ansprüche,
**dadurch gekennzeichnet, dass**
die Abmessung des Zoomobjektivs in Längsrichtung maximal 20 mm und dessen Durchmesser maximal 10 mm beträgt.

6. Zoomobjektiv vorzugsweise für Endoskopiegeräte gemäß einem der vorstehenden Ansprüche, des Weiteren mit:
einem optischen Sensorchip (14) zum Umwandeln der aufgenommenen optischen Informationen in elektrische Signale.

7. Zoomobjektiv vorzugsweise für Endoskopiegeräte gemäß Anspruch 4, des Weiteren mit:
einer Vielzahl von Zylindern (19 - 21),
einer Vielzahl von Kolben (22 - 24), welche jeweils in den Zylindern (19-21) verschiebbar angeordnet sind,
wobei die Innenflächen der Zylinder (19 - 20) und die in die Zylinder (14- 21) eingefügten Außenflächen der Kolben (22 - 24) jeweils Aktuatorkammern (31 - 33) ausbilden, welche über die Vielzahl von Fluidleitungen (16-18) mit den Druckräumen (7 - 9) in Verbindung stehen.

8. Zoomobjektiv vorzugsweise für Endoskopiegeräte gemäß Anspruch 7,
**dadurch gekennzeichnet, dass**
ein Außendurchmesser der Kolben (22 - 24) wesentlich kleiner als ein Innendurchmesser der Zylinder (19-21) ist.

9. Zoomobjektiv vorzugsweise für Endoskopiegeräte gemäß Anspruch 7 oder 8,
**dadurch gekennzeichnet, dass**
jeweils ein Kolben (22 - 24) mittels eines Elektro-Schrittmotors bewegt wird.

10. Zoomobjektiv vorzugsweise für Endoskopiegeräte gemäß Anspruch 9, des Weiteren mit:
einer Vielzahl an Untersetzungseinheiten, welche zwischen dem Elektro-Schrittmotor und dem Kolben (22, 23, 24) zur Leistungsübertragung angeordnet sind.

11. Zoomobjektiv vorzugsweise für Endoskopiegeräte gemäß
einem der Ansprüche 3 bis 6, des Weiteren mit:
einer Vielzahl an Balgen (119-121), deren Innenräume jeweils über die Vielzahl an Fluidleitungen (16-18) mit den Druckräumen (7 - 9) in Verbindung stehen und
einer Vielzahl an Schäften (122 - 124), welche jeweils an einem Ende der Balgen (119-121) angeordnet sind, um durch Bewegen eines Schafts (122 - 124) den jeweiligen Balg (119 - 121) auszudehnen oder zusammenzudrücken.

12. Zoomobjektiv vorzugsweise für Endoskopiegeräte gemäß Anspruch 11,
**dadurch gekennzeichnet, dass**
jeweils ein Schaft (122 - 124) mittels eines Elektro-Schrittmotors bewegt wird.

13. Zoomobjektiv vorzugsweise für Endoskopiegeräte gemäß Anspruch 12, des Weiteren mit:
einer Vielzahl an Untersetzungseinheiten, welche jeweils zwischen dem Elektro-Schrittmotor und dem Schaft (122 - 124) zur Leistungsübertragung angeordnet sind.

## Claims

1. A zoom lens preferably for endoscopic devices comprising:
a lens mount (13),
a plurality of lens units (1 - 4), each consisting of at least one lens (28 - 30), which are arranged in said lens mount (13) at an axial distance from each other, wherein a focal length of said zoom lens is variable, by hydraulically and/or pneumatically varying the distance between said lens units (1-4), for which purpose between each respective neighboring lens units (1 - 4), a pressure chamber is formed for hydraulically/pneumatically varying the focal length whereby pressure chambers (7 - 9) are formed at both sides of said lens units (2, 3), which are adjustable, **characterized in that** a spring element (10- 12) is arranged between said lens units (1 - 4) in each of the respective pressure chambers, being biased in a specific manner to position said lens units (2, 3), which are adjustable, in a home or constructional position.

2. A zoom lens preferably for endoscopic devices according to one of the preceding claims,
**characterized in that**
at least one of said lens units (2, 3) is hydraulically movable in an axial direction of said lens mount (13) so as to vary the focal length of said zoom lens.

3. A zoom lens preferably for endoscopic devices according to one of the preceding claims, **characterized in that** between two respective neighboring lens units (1 - 4) fluid conduits (16-18) are connected to the pressure chambers (7 - 9).

4. A zoom lens preferably for endoscopic devices according to claim 3,
**characterized in that**
a variation of the pressure difference between the pressure chambers (7 - 9) for adjusting the focal length of said zoom lens is effected by supplying fluid to selected pressure chambers (7 - 9) or discharging fluid from other selected pressure chambers (7-9).

5. A zoom lens preferably for endoscopic devices according to one of the preceding claims,
**characterized in that**
a maximum dimension of said zoom lens in a longitudinal direction is 20 mm and a maximum diameter thereof is 10 mm.

6. A zoom lens preferably for endoscopic devices according to one of the preceding claims, further comprising:
an optical sensor chip (14) for converting received optical information to electric signals.

7. A zoom lens preferably for endoscopic devices according to claim 4, further comprising:
a plurality of cylinders (19 - 21),
a plurality of pistons (22 - 24) each being movably arranged in said cylinders (19 -21),
wherein inner surfaces of said cylinders (19 - 20) and outer surfaces of said pistons (22 - 24) inserted in said cylinders (14 - 21) form respective actuator chambers (31 - 33) which are communicated with said pressure chambers (7 - 9) through a plurality of said fluid conduits (16-18).

8. A zoom lens preferably for endoscopic devices according to claim 7 or 8,
**characterized in that**
an outer diameter of said pistons (22 - 24) is considerably smaller than an inner diameter of said cylinders (19-21).

9. A zoom lens preferably for endoscopic devices according to claim 7,
**characterized in that**
one piston (22 - 24) at a time is moved by means of an electric step motor.

10. A zoom lens preferably for endoscopic devices according to claim 9, further comprising:
a plurality of reduction units arranged between said electric step motor and said piston (22, 23, 24) respectively for power transmission.

11. A zoom lens preferably for endoscopic devices according to one of claims 3 to 6, further comprising:
a plurality of bellows (119-121) having interiors which are communicated with said pressure chambers (7 - 9) via the plurality of said respective fluid conduits (16-18), and
a plurality of shafts (122 - 124) each of which is arranged at one end of said bellows (119 - 124) so as to expand or compress said respective bellow (119 - 124) by moving a shaft (122 -124).

12. A zoom lens preferably for endoscopic devices according to claim 11,
**characterized in that**
one shaft (122 - 124) at a time is moved by means of an electric step motor.

13. A zoom lens preferably for endoscopic devices according to claim 12, further comprising:
a plurality of reduction units being arranged between said electric step motor and said shaft (122 - 124) respectively for power transmission.

## Revendications

1. Objectif zoom, de préférence pour des appareils endoscopiques, comportant :
une monture pour lentilles (13),
un nombre d'unités de lentilles (1 - 4) comprenant chacune au moins une lentille (28 - 30), lesquelles sont disposées dans la monture pour lentilles (13) à distance axiale les unes des autres, la distance focale de l'objectif zoom étant réglable en modifiant par voie hydraulique et/ou pneumatique la distance entre les unités de lentilles (1 - 4), c'est pourquoi une chambre sous pression pour la variation par voie hydraulique/pneumatique de la distance focale est réalisée entre des unités de lentilles (1 - 4) adjacentes, moyennant quoi des chambres sous pression (7 - 9) sont réalisées dans chaque cas de part et d'autre des unités de lentilles (2, 3) qui sont réglables, **caractérisé en ce que** dans chacune des chambres sous pression entre les unités de lentilles (1 - 4) est agencé un élément à ressort (10 - 12) qui est maintenu sous une précontrainte déterminée, afin de positionner dans une position initiale ou position de montage les unités de lentilles (2, 3) qui sont réglables.

2. Objectif zoom, de préférence pour des appareils endoscopiques, selon l'une des revendications précédentes, **caractérisé en ce qu'**au moins une des unités de lentilles (2, 3) est mobile par voie hydraulique dans la direction axiale de la monture pour lentilles (13), afin de modifier ainsi la distance focale de l'objectif zoom.

3. Objectif zoom, de préférence pour des appareils endoscopiques, selon l'une quelconque des revendications précédentes, **caractérisé en ce que** des conduites de fluide (16 - 18) sont raccordées aux chambres sous pression (7 - 9) entre les unités de lentilles (1 - 4) adjacentes.

4. Objectif zoom, de préférence pour des appareils endoscopiques, selon la revendication 3, **caractérisé en ce qu'**une variation de la différence de pression entre les chambres sous pression (7 - 9) pour le réglage de la distance focale de l'objectif zoom est produite par le fait que le fluide est acheminé dans des chambres sous pression (7 - 9) sélectionnées ou est évacué hors d'autres chambres sous pression (7 - 9) sélectionnées.

5. Objectif zoom, de préférence pour des appareils endoscopiques, selon l'une quelconque des revendications précédentes, **caractérisé en ce que** la dimension de l'objectif zoom dans le sens longitudinal est de l'ordre de 20 mm maximum et le diamètre de celui-ci est de l'ordre de 10 mm maximum.

6. Objectif zoom, de préférence pour des appareils endoscopiques, selon l'une quelconque des revendications précédentes, comportant en outre :
une puce de détection (14) optique pour transformer les informations optiques reçues en signaux électriques.

7. Objectif zoom, de préférence pour des appareils endoscopiques, selon la revendication 4, comportant en outre :
une pluralité de cylindres (19 - 21),
une pluralité de pistons (22 - 24), qui sont agencés chacun de manière mobile dans les cylindres (19 - 21),
les faces intérieures des cylindres (19 - 20) et les faces extérieures des pistons (22 - 24), insérées dans les cylindres (14 - 21), constituant respectivement des chambres d'actionnement (31 - 33) qui sont reliées aux chambres sous pression (7 - 9) via la pluralité de conduites de fluide (16 - 18).

8. Objectif zoom, de préférence pour des appareils endoscopiques, selon la revendication 7, **caractérisé en ce qu'**un diamètre extérieur des pistons (22 - 24) est sensiblement plus petit qu'un diamètre intérieur des cylindres (19 - 21).

9. Objectif zoom, de préférence pour des appareils endoscopiques, selon la revendication 7 ou 8, **caractérisé en ce que** respectivement un piston (22 - 24) est déplacé au moyen d'un moteur pas à pas électrique.

10. Objectif zoom, de préférence pour des appareils endoscopiques, selon la revendication 9, comportant en outre :
une pluralité d'unités de démultiplication qui, en vue de la transmission de la puissance, sont disposées entre le moteur pas à pas électrique et le piston (22, 23, 24).

11. Objectif zoom, de préférence pour des appareils endoscopiques, selon l'une quelconque des revendications 3 à 6, comportant en outre :
une pluralité de soufflets (119 - 121), dont les volumes intérieurs communiquent respectivement avec les chambres sous pression (7 - 9) via la pluralité de conduites de fluide (16 - 18), et
une pluralité de tiges (122 - 124), qui sont disposées chacune à une extrémité des soufflets (119 - 121) afin de dilater ou de comprimer le soufflet (119 - 121) concerné au moyen du déplacement d'une tige (122 - 124).

12. Objectif zoom, de préférence pour des appareils endoscopiques, selon la revendication 11, **caractérisé en ce que** respectivement une tige (122 - 124) est déplacée au moyen d'un moteur pas à pas électrique.

13. Objectif zoom, de préférence pour des appareils endoscopiques, selon la revendication 12, comportant en outre :
une pluralité d'unités de démultiplication qui, en vue de la transmission de la puissance, sont disposées respectivement entre le moteur pas à pas électrique et la tige (122 - 124).
